# EUROPEAN PATENT SPECIFICATION

(11) **EP 0 599 990 B1**
(45) Date of publication and mention of the grant of the patent: **24.01.2001**
(21) Application number: 92918534.6
(22) Date of filing: 12.08.1992
(51) Int. Cl.: G01N 33/543, G01N 33/577, G01N 33/569

(54) **HLA TYPING**
HLA-TYPISIERUNG VON HUMANEN LEUKOZYTEN
TYPAGE D'ANTIGENES DE LEUCOCYTES HUMAINS (ALH)

(30) Priority: 15.08.1991 US 745163
(43) Date of publication of application: 08.06.1994
(73) Proprietor: SANGSTAT MEDICAL CORPORATION, Menlo Park, CA 94025 (US)
(72) Inventor: POULETTY, Philippe, Atherton, CA 94025 (US); CHUN, Peter, South San Francisco, CA 94080 (US); CHANG, Chin, Hai, Los Altos, CA 94024 (US)
(74) Representative: O'Brien, Caroline Jane
(86) International application number: US9206787
(87) International publication number: WO9304372

(56) References cited:
- WO-A-87/05398
- WO-A-92/21030
- US-A- 4 895 706
- US-A- 4 937 199
- US-A- 5 098 827
- TISSUE ANTIGENS, vol. 42, 1993, COPENHAGEN DK, pages 14-19, XP000569123 P. POULETTY ET AL.: "Typing of serum-soluble HLA-B27 antigen by ELISA. "
- Human Immunology, Vol. 21, issued 1988, K. SAKAGUCHI et al., "Anti-HLA-B7,B27,Bw42,Bw54,Bw55,Bw56,Bw67,B w73monoclonalantibodies; Specificity, idiotypes, and application for a double determinant immunoassay", pages 193-207, see Figures 4-5 and Tables 6-7.
- Russo et al, Human Immunology vol. 19, 1987, p. 69 - 77

## Description

### INTRODUCTION

### Technical Field

The technical field of this invention is HLA typing.

### Background

There is substantial interest in being able to type human leukocyte antigens (HLAs) for a variety of reasons. In many situations it has been found that specific HLA alleles may be associated with a susceptibility to a particular disease. For example, HLA-B27 has been associated with ankylosing spondylitis and related diseases. When transplanting organs to a host it is desirable that the organs be matched, so as to minimize the risk of rejection. HLA typing may also find application in determining lineage, epidemiology and the like.

There is an extensive family of HLA antigens divided into Class I and Class II. In each of the classes, there are polymorphic regions. These sites may or may not provide for epitopes which will induce an immune response which will allow for the preparation of antisera or monoclonal antibodies which are specific for a specific HLA allele and able to distinguish that HLA allele from other HLA allele.

This situation is exemplified by the cross-reactivity between HLA-B27 and HLA-B7 where monoclonal antibodies are not readily available which are specific for HLA-B27, so as not to cross-react with HLA-B7 or other HLA allele.

Since mammals are diploid, there will be pairs of HLA antigens as to each of the particular groups. Thus, unless one can determine specifically a particular HLA allele, one cannot be certain whether there are two different alleles or one is observing cross-reactivity. There is, therefore, substantial interest in developing methods which will allow for the accurate detection of a particular HLA allele, where substantial cross-reactivity is observed with other HLA alleles.

### Relevant Literature

Sakaguchi et al., Human Immunology, 21:193-207 (1988) describes use of monoclonal antibodies in determination of HLA-B27 and a double determinant immunoassay for detection of HLA-B27. Villar et al., Eur. J. Immunol. 19:1835-39 (1989) describe the detection of Class I molecules from a variety of sources. Toxiadis and Grosse-Wilde, Vox Sang 56:196-99 (1989) describe the detection of HLA Class I proteins.- Ferreira et al., Clin. Chim. Acta. 174:207-11 (1988) describe the use of a solid-phase enzyme immunoassay for detection of HLA Class I antigens in sera.

WO-A-92/21030 describes a method in which physiological samples are typed for HLA-B27. The method involves contacting the sample with an antibody which binds to HLA-B7 and is not cross-reactive between HLA-B7 and B27, separating the sample from any complexes which are formed and then testing the sample for the presence of HLA-B27 with an antibody which is cross-reactive for HLA-B27 and B27 in a STAT test.

WO-A-87/05398 also describes a method in which samples are tested for HLA-B27. The method involves mixing blood cells or blood cell lysates with a first antibody capable of binding and blocking the HLA-B7 antigen and a detectably labelled second antibody capable of binding to the HLA-B27 antigen and thereafter detecting the second antibody. The second antibody is not specific to the HLA-B27 antigen. It is taught that the HLA-B7 antigen will bind in significant quantities to the second antigen and that it is necessary to block the HLA-B7 antigen with a first antibody.

US 4937199 describes a method for detecting a herpes virus or antibody to such a virus in a clinical sample containing beta₂m. The method involves assaying the sample with a reagent that will reveal the presence of the virus or antibody and utilising or removing the beta₂m prior to the assay.

### SUMMARY OF THE INVENTION

Methods are provided for detecting closely related alleles employing immunoassays, where there is no available antibody to distinguish between two alleles. The method finds specific exemplification in relation to distinguishing the HLA alleles B27 and B7, where B27 is of diagnostic interest. The invention is exemplified by a sandwich assay where separate wells have a cross-reactive antibody in one well and an antibody specific for one of the alleles in the other well. By appropriate use of controls and measurement of signals in the different wells, a cutoff value is determined which allows for the accurate determination of the presence or absence of the allele.

### DESCRIPTION OF THE SPECIFIC EMBODIMENTS

Methods are provided for the accurate detection of one of two polymorphic antigens in a sample, where the antigens are characterized by having no useful receptor readily available to distinguish between the two antigens, the antigens are members of a much larger group of antigens, usually substantially in excess of 10, where there is substantial cross-reactivity between the antigens in sharing numerous epitopes, and differences between the antigens may be subtle, involving only one or a few amino acids.

WO-A-92/21030 which is relevant under the provisions of Art: 54/3) EPC only employs a separation, where an antibody which binds in a concentration range to only one but not both of the alleles is employed for removing the complementary allele from the sample. The cross-reactive monoclonal antibody, which cross-reacts with the two alleles, may then be used to determine the allele for which a specific antibody is not available.

In a sandwich immunoassay exemplifying the invention, where one employs negative and positive controls, as well as two additional wells, one well having the cross-reactive antibody and the other well having an antibody which reacts with only one of the two cross-reactive alleles. One then adds labeled antibody which is cross-reactive with both of the alleles, and obtains values of the level of label present in the various wells for the two controls and the two sample wells. Using the values obtained, one obtains a cutoff value to compare the value in the well with the antibody which is cross-reactive with the two alleles. Values for the sample above the cutoff value indicate the presence of the particular allele.

The separation methodology broadly as in WO-A-92/21030 will be discussed here. The methodology employs as a first stage, using a binding moiety, usually a monoclonal antibody, to specifically deplete HLA-B7 antigen from a sample to a non-interfering level, particularly a blood sample or sample derived therefrom. The resulting HLA-B7 depleted medium is then combined with a second binding moiety, usually a monoclonal antibody, which has a high affinity for B27 and B7. Formation of immune complexes between the binding moiety and any B27 present is detected as indicating the presence of soluble B27 in the sample.

In such a methodology involving a depletion step, any binding moiety binding to the target antigen(s) or having the requisite discrimination as to the different HIA alleles may be used. Binding moieties include antibodies, any of the isotypes, e.g., IgG, IgM, etc., fragments thereof, e.g. Fab, F(ab')₂, Fv, etc., binding peptides, T-cell receptors, or the like. For the most part, monoclonal antibodies are the most convenient and exemplify the problem of cross-reactivity, where the available monoclonal antibodies in relation to HLA-B27 are unable to specifically distinguish this allele from other alleles. Other binding moieties having analogous cross-reactivities may be substituted.

Whether using a methodology as in WO-A-92/21030 or a method as described herein, the sample, normally blood, serum, plasma or urine, may be subjected to prior treatment such as dilution in buffered medium, concentration, filtration, or other gross treatment which will not involve any specific separation. The sample can be relatively small, generally being not less than about 1 µl and will generally not exceed about 500 µl, generally being in the range of about 10 to 200 µl. Dilution will usually not be more than 5-fold, more usually not more than 2-fold and concentration will normally not be necessary.

The sample is contacted with an antibody which is reactive with HLA-B7, but not significantly reactive with HLA-B27, but may be cross-reactive with any other HLA allele. The significant factor for the antibody is that it can distinguish between HLA-B7 and HLA-B27. While many antibodies which ostensibly have this capability, may not prove to be satisfactory, there are a number of antibodies in the ATCC catalog, as well as catalogues of other repositories, which are satisfactory. As appropriate, purified polyclonal antisera may be employed, where the antisera is prepared in response to HLA-B7 and then purified to ensure that any antibodies cross-reactive between HLA-B7 and HLA-B27 are removed. This can be achieved by passing the antisera through an affinity column comprising HLA-B27 randomly attached to a support, such as agarose, Sephadex, polystyrene beads, magnetic beads, nylon membranes or the like. The eluent may then be assayed for its ability to bind to soluble HLA-B27.

In carrying out a methodology as described in WO92/21030, the sample may be passed through an affinity column comprising anti-HLA-B7 bound to a support, may be mixed with anti-HLA-B7, directly or indirectly, covalently or non-covalently bound to beads or particles, e.g., magnetic particles, may be mixed with anti-HLA-B7 and contacted with, in a column, microtiter well, or other container, anti-mouse (or other species) Ig antibody bound to a support, where the anti-HLA antibody is a mouse (or other species) antibody, or may be mixed with anti-HLA-B7, conjugated with a ligand, e.g., biotin, followed by contacting with avidin, or streptavidin ("strept/avidin") bound to a support as described above. (By "directly or indirectly" is intended that the molecule in question e.g., anti-HLA-B7, is either directly bound to the solid support e.g., covalently through a spacer arm or is bound to an intermediate molecule directly bound to the solid support, e.g., an antibody to the molecule in question.) In each situation, the mixture is contacted or incubated for sufficient time with sufficient amount of the anti-HLA-B7 to provide for substantial complex formation of the anti-HLA-B7 and separation of the HLA-B7-anti-HLA-B7 complex from the sample medium.

An affinity column employing a receptor bound to a support, conveniently a polysaccharide, more particularly Sepharose may be used. The Sepharose may be activated by any convenient means, such as cyanogen bromide, carbodiimide, bromoacetyl, p-carboxyphenacyl bromide, or the like. The receptor may then be conjugated to the support in accordance with conventional ways, depending upon the nature of the functionality bound to the support. Cyanogen bromide and other active halides do not require any activation, while carboxyl groups may be activated with carbodiimide. After reacting the activated support with the antibody, any unreacted functionalities may be terminated with an alkanolamine, e.g., ethanolamine. The amount of antibody will generally be from about 0.2-2 mg/0.5 ml of packed gel.

Alternatively, 1-5 µ magnetic beads coated with anti-HLA-B7 antibody (10⁶-10⁷ beads) are incubated with a serum or other specimen e.g., 100 µl, for 5-30 min at ambient conditions. The beads may be separated using a magnetic filter, a solenoid or the like.

As set forth in WO-A-92/21030, once the sample has been at least substantially depleted of any HLA-B7 which may have been present, the sample may now be assayed for the presence of B27. Various assays may be employed for detection of the presence of HLA-B27. A number of STAT assay protocols may be employed, as desired. A suitable STAT assay employs a porous filter having measuring regions which are separated by nonporous regions. The filter or membrane is supported by an absorbent layer, which serves to absorb the sample and provides for flow of the sample through the membrane. Desirably, the membrane and absorbent layer are separated by a flow control layer, which may assume various characteristics. Depending upon the nature of the membrane, the membrane may be coated on the fluid exiting side with a coating which will substantially reduce the pore size of the membrane.

Various membranes may be employed to carry out a methodology eg as set forth in WO-A-92/21030, although it is found that glass fiber membranes and nylon membranes appear to be substantially superior to other types of membranes. Conveniently, the glass membrane may be sprayed with an acrylic polymer, which does not interfere with the assay procedure, but provides for the desired flow rate. The amount of acrylic polymer which is applied may be determined empirically, although generally the amount will be in the range of about 0.1 to 1 mg/cm² with a glass membrane having a pore size in the range of about 0.2 to 5 µ. The separation of the various measurement regions may be achieved by using non-porous tape or other convenient barrier. In addition, strips may be provided over the barrier to provide a visual indication of the areas in the different regions.

For each sample, there will be desirably three regions having capture antibodies: (1) a procedural positive region coated with an anti-immunoglobulin antibody specific for the antibody conjugate which binds to the HLA-B27 or coated with an anti-Ig where the Ig is of the isotype of the anti-HLA-B27; (2) a test region coated with a monoclonal antibody which is cross-reactive with HLA-B27 and HLA-B7, but not cross-reactive with other HLAs; and (3) a negative procedural region coated with the same anti-HLA-B7 monoclonal antibody as used for depleting HLA-B7. A specimen which is HLA-B27 positive shows color development in the first two wells but not the third; a specimen which is HLA-B7 positive and HLA-B27 negative, where the HLA-B7 has been properly depleted or a HLA-B7/B27 negative specimen, would only show a positive result in the first region; and a specimen which is B7 positive and has not been properly absorbed will be positive in all three regions.

The methodology of the present invention has similarities to the aforementioned methodology as generally discussed above with reference to WO-A-92/21030 and may use similar reagents (see above discussion).

The method employs two series of sample wells, a first series using as the capture antibody the cross-reactive antibody and a second series using as the capture antibody the antibody which binds to only one of the two alleles, but may bind to other alleles. The specimens may be whole blood from which the serum is separated, plasma specimens containing heparin, EDTA or ACD as an anti-coagulant, cerebral spinal fluid ("CSF") specimens, or cell culture supernatant specimens. The specimens may be stored undiluted up to 24 h at room temperature, one week between 2-8°C, frozen at -20°C or below, if longer storage is required.

Various devices may be used in which to carry out the assay, such as strips, microtiter well plates, slides, test tubes, STAT cartridges, and the like. Since small volumes will be used and repetitive washes will be employed, the devices employed should allow for concentration of the reagent providing the detectible signal as well as absorption or removal of washed solutions.

The materials employed will include the device comprising a plurality of wells or spots which have one or the other antibody bound thereto in a predetermined amount. The amount should be sufficient to bind enough of the allele to be determined to allow for detection of the allele. This can be readily determined empirically. A positive control is provided, which comprises the allele in an appropriate buffer at an appropriate concentration in relation to the amount of allele which would be expected to be present in a positive sample. For example, with B27 allele, the amount of B27 which is present will range from about 30 ng/ml to 1µg/ml. A negative control will be provided, which will be buffer.

To carry out the assay, one may add the positive control and the negative control to one or more wells or sites with each of the antibodies. Desirably, one will have two wells or sites for each determination, so that an average value can be determined for the positive and negative controls. The specimen may then be added to one or more wells with each of the antibodies. Generally, the specimen volume will be in the range of about 20 to 200 µl, more usually about 20 to 100 µl, where the specimen will normally be used neat or prediluted in a buffered solution. The sample is then incubated for sufficient time to ensure complete binding. Usually, the incubation will be at least about 15 min, more usually at least about 30 min and generally not more than about 6 h, usually not more than about 4 h. For the most part, 2 h has been found to be adequate. The incubation may be carried out at ambient temperatures. At the end of the incubation, any liquid present is removed and the wells or sites thoroughly washed, conveniently with deionized water. Usually, at least about two washes will be used, where the total number of washes may be five or more. Where wells are employed, the well may be filled with the wash solution, allowed to stand, then decanted and the process repeated.

A labeled conjugate may then be added to the wells or sites. The label may be an enzyme, radioisotope, fluorescer, chemilumenescer, etc. Desirably, the label will be an enzyme. The antibody will be specific for an epitope common to the alleles. In the case of Class I HLA, the antibody may be conveniently to β2-microglobulin. Alternatively, the antibody could be to the constant region, or in the case of two alleles, to a common epitope, other than the epitope to which the capture antibody binds. Again, the assay mixtures may be incubated for sufficient time for binding, usually at least 0.5 h, and not more than about 3 h, 1.5 h usually sufficing. Ambient temperatures may be employed. The complexes of antibody and antigen are then thoroughly washed, similarly to the wash procedure used previously.

With an enzyme conjugate, a substrate solution is then added to the wells or sites and the reaction allowed to proceed for a predetermined time, generally at least about 5 min and not more than about 1 h. A stop solution may then be added, which terminates the reaction, without being detrimental to the detection of the dye which has formed from the substrate. Depending upon the nature of the enzyme product or other label which is present, one may then determine the amount of detectable label or product which is present. One may use spectrophotometers, fluorimeters, light scattering devices, or the like.

One then analyzes the results as follows. The negative controls must be below a predetermined cutoff value, which can be determined empirically. The positive controls should also fall within a predetermined range, based on what would be expected for the known amount of allele present. The ratio of the values between the cross-reactive antibody and the single allele antibody will also be required to be within a predetermined range. If all of these values are found to be within the required ranges, then one may proceed with determining whether the allele of interest is present.

An empirical discrimination value is then determined which is, as exemplary, the signal value of the negative control obtained with the cross-reactive antibody plus a percentage, e.g. 20%, of the positive control value obtained with the cross-reactive antibody. If the observed value of the signal with the specimen with the cross-reactive antibody is less than the discrimination value, the result is negative. If the value is about equal to or greater than the discrimination value, one proceeds to calculate the cutoff for a positive result by multiplying the ratio of the value of the positive control with the cross-reactive antibody divided by the value of the positive control with the single allele antibody multiplied by an empirical factor, e.g., two. If the ratio of the value of the specimen with the cross-reactive antibody to the specimen with the single allele antibody is equal to or greater than the cutoff which has been determined, the result is considered positive, but if the ratio is less, the result is considered negative.

Paper can be supplied having the appropriate legend or a simple algorithm, which will allow for the rapid determination of a positive or negative result. Thus, the reading device may be connected with a computer, which will allow for a direct readout, without requiring any manual computation.

The following examples are offered by way of illustration and not by way of limitation.

### EXPERIMENTAL

### Example 1: Exemplifying Invention of WO92/21030 HLA B27 Test

1. To 100 µl of patient serum add 1-4 µl of 0.5 mg/ml anti-B7 antibody which cross-reacts with HLA-B7 and HLA-B40, but does not react with HLA-B27 (Incstar Corporation). Vortex and incubate at room temperature for 5-10 minutes.
2. Add 200-600 µl of magnetic beads coated with goat anti-mouse IgG (Advanced Magnetics, Inc., Catalog No. 4340D) to the above mixture. Cap the tube and mix on a rocker at room temperature for 20-30 minutes.
3. Place the tube on the surface of a strong magnet in upright position for 2-4 minutes to completely bring down all magnetic beads.
4. Transfer 200 µl of the supernatant to the STAT cartridge (SangStat Medical Corp., Menlo Park, CA). Allow to drain completely. [STAT cartridge containing 3 miniwells (A, B, C) with nylon membrane coated with antibodies at a concentration of 1 mg/mL in 0.1 M PBS pH 7.2. Miniwell A coated with goat anti-mouse antibody (Jackson Laboratories) diluted 1:50 in normal goat serum. Miniwell B coated with the anti-HLA B7/B27 monoclonal antibody (Clone SV90.1). Miniwell C coated with the HLA B7/B40 specific monoclonal antibody (Incstar Corporation)].
5. Add 200 µl of antibody conjugate (anti-beta-2 microglobulin goat antibody conjugated to alkaline phosphatase, diluted to 5 µg/mL in 0.1 M PBS pH 7.2 containing 1% casein an 0.1% Tween 20) to the STAT cartridge. Allow to drain completely.
6. Add 1 mL of wash solution (0.1 M PBS pH 7.2 with 0.1% Tween 20) to each cartridge. Allow to drain completely.
7. Add 200 µl of chromogenic substrate (BCIP/NBT substrate solution) (Sangstat Medical Corporation). Allow to develop for 3-4 minutes.
8. Read results.

### INTERPRETATION OF TEST RESULTS

The development of blue-grey rings in the first miniwell only (miniwell A) is a negative test for HLA-B27.

The development of blue-grey rings in the first (A) and second (B) miniwells, with the third (C) miniwell showing no rings or lighter rings than in the second (B) miniwell, is a positive test for HLA-B27.

### QUALITY CONTROL

If there is no color development in the first miniwell (Miniwell A, positive procedural reference) or uniform color development in all miniwells, the test is invalid.

Color development in miniwell C indicates that removal of HLA-B7 (or B40) was incomplete or that there is an interference which may be related to anti-mouse IgG antibodies in the serum specimen.

### RESULTS

Thirty specimens collected from individuals previously phenotyped by microcytotoxicity assay were tested by the rapid assay (HLA-B27).

| CYTOTOXICITY | | | | |
|---|---|---|---|---|
| | B27+/B7+ | B27+/B7- | B27-/B7+ | B27-/B7- |
| HLA B27 STAT | | | | |
| Positive | 4 | 10 | 0 | 0 |
| Negative | 0 | 0 | 5 | 15 |
| Invalid | 0 | 0 | 1 | 0 |

The immunoglobulins may be as one or more circles, where there is a concentration gradient from the center outward. One can have a relatively high immunoglobulin concentration central region and a substantially lower concentration outer periphery. The concentration gradient can be readily achieved by using a porous pointed stub which is pressed against the membrane, so that a significant proportion of the immunoglobulin present in the sample becomes bound around the stub point creating a relatively high concentration in a central region and a substantially lower concentration around the central region.

The rate of flow of the sample through the membrane will provide for at least about 10 to 120 sec for contact of the sample with the antibody on the membrane. Similarly, the rate of flow and volume of the enzyme conjugate will allow for 10 to 120 sec of contact with the enzyme conjugate in the various regions. Finally, the rate of flow and volume of the substrate will allow for 10 to 120 sec of reaction of the enzyme with the substrate.

Kits for carrying out a depletion method as set forth by W092/21030 may have a STAT test cartridge having the three regions described above, the packing for the column, the enzyme conjugate, and, as appropriate, other reagents such as wash solutions, enzyme substrate and the like.

### Example 2: Exemplifying Present Invention

A kit is employed which comprises a strip of wells coated with monoclonal antibody (KS4) which cross-reacts with HLA-B27 and HLA-B7, and a second strip which is coated with monoclonal antibody (NB40), that reacts with HLA-B7, but not HLA-B27. A positive control containing 1.26 µg/ml of monomorphic HLA-B7 in RPMI/BSA buffer and a negative control (buffer) are provided. The controls and specimens are placed in wells of each of the strips at the same time and allowed to incubate for 2 h at room temperature.

After the incubation, the solutions are aspirated from each of the wells, and each of the wells is washed by filling the well with deionized water, emptying the well and repeating the wash cycle five times. After aspirating the well for the last time, 100 µl of an HRP-(anti-β₂ microglobulin antibody) conjugate (excess antibody) is added to each well. After incubating for 90 min at room temperature, the conjugate solution is aspirated from each of the wells, the wells washed as above and 100 µl of 1 mg o-phenylene diamine in buffer is added to each solution and the reaction allowed to proceed for 30 min. The reaction is kept in the dark. At the end of the reaction, 100 µl of a stop solution (1N HCl) is added. The absorbance of each well at 490-495 nm is read within 5-8 min after adding the stop solution.

### Interpretation of assay results

### Definitions:

| | | |
|---|---|---|
| Black strips = B27/B7 assay | | |
| Clear strips = B7 assay | | |
| A = Absorbance value (490-498 nm) | | |
| PC = Positive | PCl = Positive | APCl = Absorbance value |
| control | control | Positive control |
| | B7/B27 assay | B7/B27 assay |
| | | |
| NC = Negative | NC1 = Negative | ANC1 = Absorbance value |
| control | control | Negative control |
| | B7/B27 assay | B7/B27 assay |
| | | |
| PC = Positive | PC2 = Positive | APC2 = Absorbance value |
| control | control | Positive control |
| | B7 assay | B7 assay |
| NC = Negative | NC2 = Negative | ANC2 = Absorbance value |
| control | control | Negative control |
| | B7 assay | B7 assay |
| | | |
| A1 = Absorbance value (mean value) of a specimen in the B7/B27 assay | | |
| | | |
| A2 = Absorbance value (mean value of a specimen in the B7 assay | | |
| | | |
| A1/A2 = Specimen Absorbance ratio = Absorbance value (mean value) in the B7/B27 assay divided by Absorbance value (mean value) in the B7 assay | | |

(1) Determining mean absorbance values:
   Average the duplicate absorbance (A) for the Positive Control (PC), Negative Control (NC) and each specimen in respective assays (the B7/B27 assay [A1] and the B7 assay [A2] individually). If any specimen shows A > 2.0 in the B7 assay, it must be tested again in both assays after predilution (1:2) with the Negative Control.
(2) Validate the assay run:
   B7/B27 assay: ANC1 must be less than 0.04 and APCl must be between 0.1-0.7.
   B7 assay: ANC2 must be less than 0.04 and APC2 must be between 0.5-2.0.
   Calculate the ratio APC1/APC2
   APC1/APC2 must be between 0.1-0.6.
(3) Calculate the Discrimination Value of the B7/B27 assay (DV1):
   DV1 = ANC1 + 20% APC1
(4) Compare the absorbance of each specimen in the B7/B27 assay (A1) with DV1:
   If A1 < DV1, the specimen is HLA-B27 negative
   If A1 ≥ DV1, proceed with step (5)
(5) Calculate: APC1/APC2:
   Calculate the B27 cutoff: B27 cut-off = 2 x APC1/APC2
   Calculate the absorbance ratio (A1/A2) for each specimen
   Compare A1/A2 to the B27 cut-off:
   If A1/A2 ≥ B27 cut-off, the specimen is HLA-B27 positive
   If A1/A2 < cut-off, the specimen is HLA-B27 negative

In a survey of 110 patient specimens composed of various phenotypes, it was demonstrated that the ratio of the absorbance of the values obtained with the B7/B27 antibody and the B7 antibody is a uniquely specific parameter that can readily screen out the HLA-B27 associated phenotype with a very high degree of accuracy.

**Table**

| | sHLA-STAT™ | | | B27 | |
|---|---|---|---|---|---|
| HLA-PHENOTYPES | B27 | Positive | B | 2 | 7 |
| Negative | | | | | |
| B27+/B7- | | 28 | | | 1 |
| B27+/B7+ | | 18 | | | 0 |
| B27-/B7+ | | 0 | | | 42 |

It is evident from the above results, that the subject procedure provides for a simple effective method to accurately determine the B27 HLA type of an individual, without interference from other HLAs. Thus, a rapid diagnosis may be made of an individual's propensity to certain diseases or HLA type for forensic medicine or other purpose. The procedure is simple, does not require sophisticated equipment for performance or measurement and can be easily carried out by unsophisticated operators.

## Claims

1. A method for determining the presence of a first antigen in the potential presence of a second antigen in a physiological sample, where said first and second antigens are characterized by being members of a polymorphic group of antigens which share numerous epitopes, wherein a combination of monoclonal antibodies is used, a first antibody which is cross-reactive to both antigens, but not other common members of said group of antigens and a second antibody which binds to only said second antigen of said first and second antigens, said method comprising:
combining said sample with said second antibody and determining a first value associated with said sample and said second antibody;
combining said sample with said first antibody and determining a second value associated with said sample and said first antibody,
wherein with said sample, a value of the ratio of said second value and said first value above a cut-off value is a positive result.

2. A method according to claim 1, wherein said antigens are HLA-B7 and HLA-B27.

3. A method according to any one of the preceding claims wherein said determining is by means of a label providing a detectible signed product.

4. A method according to any one of the preceding claims wherein said label is an enzyme and said determining is by means of a colored enzymatic product.

5. A method for determining the presence in a physiological sample of HLA-B27 in the potential presence of HLA-B7, employing a first monoclonal antibody binding to HLA-B7, but not HLA-B27, and a second antibody which binds to both HLA-B27 and HLA-B7, said method comprising:
combining said sample with said second antibody and determining a first value associated with said sample and said second antibody;
combining said sample with said first antibody and determining a second value associated with said sample and said first antibody;
wherein a comparison of said first and second values with a reference value is indicative of a positive or negative result.

6. A method according to Claim 5, wherein positive and negative control values are determined as said reference value, and specific ranges are defined for each of the control values and the ratio of the control values to determine whether the assay is valid.

7. Use of a kit in a method according to any one of claims 1 to 6, for determining the presence in a physiological sample of a first antigen in the potential presence of a second antigen, where said first and second antigens are characterized by being members of a polymorphic group of antigens which share numerous epitopes, wherein a combination of monoclonal antibodies is used, a first antibody which is cross-reactive to both antigens, but not other common members of said group of antigens and a second antibody which binds to only said second antigen of said first and second antigens, said kit comprising:
a membrane comprising at least two regions: a first region to which is bound the first antibody cross-reactive with said first and second antigens, but substantially not cross-reactive with other antigens of said polymorphic group; a second region to which is bound the second antibody reactive with the second antigen but not the first antigen,
an enzyme conjugate of an antibody cross reactive with said first and second antigens.

8. Use of a kit in a method according to any one of claims 1 to 6, for determining the presence in a physiological sample of a first antigen in the potential presence of a second antigen, where said first and second antigens are characterized by being members of a polymorphic group of antigens which share numerous epitopes, wherein a combination of monoclonal antibodies is used, a first antibody which is cross-reactive to both antigens, but not other common members of said group of antigens and a second antibody which binds to only said second antigen of said first and second antigens, said kit comprising:
a plurality of wells coated with said first monoclonal antibody, a plurality of wells coated with said second monoclonal antibody and an enzyme monoclonal antibody conjugate capable of binding to an epitope common to both antigens.

9. Use of a kit according to claim 7 or claim 8 wherein said second antigen is HLA-B7 and said first antigen is HLA-B27.

## Patentansprüche

1. Verfahren zum Nachweisen der Gegenwart eines ersten Antigens in potentieller Gegenwart eines zweiten Antigens in einer physiologischen Probe, worin das erste und das zweite Antigen dadurch gekennzeichnet sind, dass sie Elemente einer polymorphen Gruppe von Antigenen sind, die zahlreiche gemeinsame Epitope aufweisen, worin eine Kombination von monoklonalen Antikörpern verwendet wird, ein erster Antikörper, der mit beiden Antigenen, nicht jedoch mit anderen gemeinsamen Elementen der Gruppe von Antigenen kreuzreaktiv ist, und ein zweiter Antikörper, der sich von erstem und zweitem Antigen nur an das zweite Antigen bindet, wobei das Verfahren Folgendes umfasst:
das Kombinieren der Probe mit dem zweiten Antikörper und das Bestimmen eines der Probe und dem zweiten Antikörper zugeordneten ersten Werts;
das Kombinieren der Probe mit dem ersten Antikörper und das Bestimmen eines der Probe und dem ersten Antikörper zugeordneten zweiten Werts,
worin für die Probe der Wert des Verhältnisses zwischen den zweiten Wert und dem ersten Wert über einem Cutoff-Wert ein positives Ergebnis ergibt.

2. Verfahren nach Anspruch 1, worin die Antigene HLA-B7 und HLA-B27 sind.

3. Verfahren nach einem der vorangegangenen Ansprüche, worin der Nachweis mittels einer Markierung erfolgt, die ein nachweisbares gekennzeichnetes Produkt liefert.

4. Verfahren nach einem der vorangegangenen Ansprüche, worin die Markierung ein Enzym ist und der Nachweis mittels eines gefärbten enzymatischen Produkts erfolgt.

5. Verfahren zum Nachweis der Gegenwart von HLA-B27 in potentieller Gegenwart von HLA-B7 in einer physiologischen Probe unter Einsatz eines ersten monoklonalen Antikörpers, der sich an HLA-B7, nicht jedoch an HLA-B27 bindet, und eines zweiten Antikörpers, der sich sowohl an HLA-B27 als auch an HLA-B7 bindet, wobei das Verfahren Folgendes umfasst:
das Kombinieren der Probe mit dem zweiten Antikörper und das Bestimmen eines der Probe und dem zweiten Antikörper zugeordneten ersten Werts;
das Kombinieren der Probe mit dem ersten Antikörper und das Bestimmen eines der Probe und dem ersten Antikörper zugeordneten zweiten Werts;
worin ein Vergleich des ersten und des zweiten Werts mit einem Bezugswert ein positives oder ein negatives Ergebnis anzeigt.

6. Verfahren nach Anspruch 5, worin positive und negative Vergleichswerte als Bezugswert ermittelt werden und spezifische Bereiche für jeden der Vergleichswerte und das Verhältnis der Vergleichswerte definiert werden, um zu ermitteln, ob der Test gültig ist.

7. Verwendung eines Sets in einem Verfahren nach einem der Ansprüche 1 bis 6 zum Nachweis der Gegenwart eines ersten Antigens in potentieller Gegenwart eines zweiten Antigens in einer physiologischen Probe, worin das erste und das zweite Antigen dadurch gekennzeichnet sind, dass sie Elemente einer polymorphen Gruppe von Antigenen sind, die zahlreiche Epitope gemeinsam haben, worin eine Kombination von monoklonalen Antikörpern verwendet wird, ein erster Antikörper, der mit beiden Antigenen, nicht jedoch mit anderen gemeinsamen Elementen der Gruppe von Antigenen kreuzreaktiv ist, und ein zweiter Antikörper, der sich von erstem und zweitem Antigen nur an das zweite Antigen bindet, wobei das Set Folgendes umfasst:
eine Membran, die zumindest zwei Bereiche umfasst: einen ersten Bereich, an den der erste Antikörper gebunden ist, der mit dem ersten und zweiten Antigen kreuzreaktiv ist, aber im Wesentlichen nicht mit anderen Antigenen der polymorphen Gruppe kreuzreaktiv ist; einen zweiten Bereich, an den der zweite Antikörper gebunden ist, der mit dem zweiten Antigen, nicht jedoch mit dem ersten Antigen reaktiv ist,
ein Enzym-Konjugat eines Antikörpers, der mit dem ersten und dem zweiten Antigen kreuzreaktiv ist.

8. Verwendung eines Sets in einem Verfahren nach einem der Ansprüche 1 bis 6 zum Nachweis der Gegenwart eines ersten Antigens in potentieller Gegenwart eines zweiten Antigens in einer physiologischen Probe, worin das erste und das zweite Antigen dadurch gekennzeichnet sind, dass sie Elemente einer polymorphen Gruppe von Antigenen sind, die zahlreiche Epitope gemeinsam haben, worin eine Kombination von monoklonalen Antikörpern verwendet wird, ein erster Antikörper, der mit beiden Antigenen, nicht jedoch anderen gemeinsamen Elemente der Gruppe von Antigenen kreuzreaktiv ist, und ein zweiter Antikörper, der sich von erstem und zweitem Antigen nur an das zweite Antigen bindet, wobei das Set Folgendes umfasst:
mehrere Näpfe, die mit dem ersten monoklonalen Antikörper beschichtet sind, mehrere Näpfe, die mit dem zweiten monoklonalen Antikörper beschichtet sind, und ein Konjugat aus Enzym und monoklonalem Antikörper, das sich an ein Epitop binden kann, das beiden Antigenen gemeinsam ist.

9. Verwendung eines Sets nach Anspruch 7 oder 8, worin das zweite Antigen HLA-B7 ist und das erste Antigen HLA-B27 ist.

## Revendications

1. Méthode pour déterminer la présence d'un premier antigène en présence potentielle d'un second antigène dans un échantillon physiologique, où lesdits premier et second antigènes sont caractérisés en ce que ce sont des membres d'une famille polymorphe d'antigènes qui partagent de nombreux épitopes, où l'on utilise une combinaison d'anticorps monoclonaux, un premier anticorps qui réagit avec les deux antigènes mais non pas avec d'autres membres communs dudit groupe d'antigènes et un second anticorps qui ne se lie qu'audit second antigène desdits premier et second antigènes, ladite méthode comprenant :
la combinaison dudit échantillon audit second anticorps et la détermination d'une première valeur associée audit échantillon et audit second anticorps ;
la combinaison dudit échantillon avec ledit premier anticorps et la détermination d'une seconde valeur associée audit échantillon et audit premier anticorps,
où avec ledit échantillon, une valeur du rapport de ladite seconde valeur et de ladite première valeur au-dessus d'une valeur de coupure est un résultat positif.

2. Méthode selon la revendication 1 où lesdits antigènes sont HLA-B7 et HLA-B27.

3. Méthode selon l'une quelconque des revendications précédentes où ladite détermination se fait au moyen d'un marqueur donnant un produit signé détectable.

4. Méthode selon l'une quelconque des revendications précédentes où ledit marqueur est une enzyme et ladite détermination se fait au moyen d'un produit enzymatique coloré.

5. Méthode pour déterminer la présence dans un échantillon physiologique de HLA-B27 en présence potentielle de HLA-B7, en employant un premier anticorps monoclonal se liant à HLA-B7 mais non pas à HLA-B27 et un second anticorps qui se lie à la fois à HLA-B27 et à HLA-B7, ladite méthode comprenant :
la combinaison dudit échantillon avec ledit second anticorps et la détermination d'une première valeur associée audit échantillon et audit second anticorps ;
la combinaision dudit échantillon audit premier anticorps et la détermination d'une seconde valeur associée audit échantillon et audit premier anticorps ;
où une comparaison desdites première et seconde valeurs avec une valeur de référence indique un résultat positif ou négatif.

6. Méthode selon la revendication 5 où des valeurs positive et négative de contrôle sont déterminée en tant que ladite valeur de référence, et des gammes spécifiques sont définies pour chacune des valeurs de contrôle et le rapport des valeurs de contrôle pour déterminer si l'essai est valable.

7. Utilisation d'un kit dans une méthode selon l'une quelconque des revendications 1 à 6 pour déterminer la présence dans un échantillon physiologique d'un premier antigène en présence potentielle d'un second antigène où lesdits premier et second antigènes sont caractérisés en ce que ce sont des membres d'un groupe polymorphe d'antigènes qui partagent de nombreux épitopes, ou une combinaison d'anticorps monoclonaux est utilisée, un premier anticorps qui réagit avec les deux antigènes mais non pas avec d'autres membres communs dudit groupe d'antigènes et un second anticorps qui ne se lie qu'audit second antigène desdits premier et second antigènes, ledit kit comprenant :
une membrane comprenant au moins deux régions : une première région à laquelle est lié le premier anticorps réagissant avec ledits premier et second antigènes mais ne réagissant sensiblement pas avec les autres antigènes dudit groupe polymorphe ; une seconde région à laquelle est lié le second anticorps réagissant avec le second antigène mais non pas avec le premier antigène,
un conjugué d'enzyme d'un anticorps réagissant avec lesdits premier et second antigènes.

8. Utilisation d'un kit dans une méthode selon l'une quelconque des revendications 1 à 6 pour déterminer la présence dans un échantillon physiologique d'un premier antigène en présence potentielle d'un second anti-gène, où lesdits premier et second antigènes sont caractérisés en ce que ce sont des membres d'un groupe poly-morthe d'antigènes qui partagent de nombreux épitopes où une combinaison d'anticorps monoclonaux est utilisée, un premier anticorps qui est réactif avec les deux antigènes mais non pas avec les autres membres communs dudit groupe d'antigènes et un second anticorps qui ne se lie qu'audit second antigène desdits premier et second antigènes, et un second anticorps qui ne se lie qu'audit second antigène desdits premier et second antigènes, ledit kit comprenant :
un certain nombre de puits enduits dudit premier anticorps monoclonal un certain nombre de puits enduits dudit second anticorps monoclonal et un conjugué enzyme anticorps monoclonal capable de se lier à un épitope commun aux deux antigènes.

9. Utilisation d'un kit selon la revendication 7 ou la revendication 8 où ledit second antigène est HLA-B7 et ledit premier antiène est HLA-B27.
